# EUROPEAN PATENT APPLICATION

(11) **EP 1 589 098 A1**
(43) Date of publication of application: **26.10.2005**
(21) Application number: 04425278.1
(22) Date of filing: 21.04.2004
(51) Int. Cl.: C12N 5/06, C12N 5/18

(54) **Tissue of animal origin for industrial uses constituted of material in sheets , in vitro processes for the production thereof**

(71) Applicant: Conciaricerca Italia S.r.l., 20123 Milano (IT); Centro di Ricerca Interdipartimentale Sui Biomateriali Università di Napoli "Federico II", 80125 Napoli (IT)
(72) Inventor: Netti, Paolo Antonio, 80100 Napoli (IT); Rossi, Mosè, Napoli (IT); Brugnoli, Federico, 20090 San Felice, Pioltello, Milano (IT); Ioannidis, Ioannis, 17563 Athens (GR); Battista, Sabrina, 80128 Napoli (IT)
(74) Representative: Faggioni, Carlo Maria

(57) **Abstract**

Bioengineered tissue of animal origin for industrial uses comprising a plurality of layers of bioengineered sheet material, obtained and interconnected through in vitro tissue-forming and/or tissue-repairing processes by eukaryote cells, such as bovine or murine fibroblasts, seeded onto bioactive scaffolds. The invention also comprises the bioengineered sheet material used as an intermediate product in the preparation of said tissue, and a process for making a bioengineered tissue in which several elements of said bioengineered sheet material are arranged adjacent in a same culture bed and are biologically interconnected through in vitro tissue-forming and/or tissue-repairing processes by eukaryote cells seeded in said culture bed.

## Description

### Field of the invention

The present invention relates to the production of bioengineered tissue of animal origin for industrial uses. The invention also relates to processes to produce such tissue through in vitro culture techniques and to the bioengineered sheet material used as an intermediate product in such processes.

Among the diverse possible industrial uses of the bioengineered tissue of the present invention for which there is currently great interest in the market, there is certainly its use as raw material for the tanning industry, in combination with or as a substitute for natural animal skins, for the production of skins for the shoe, clothing, and upholstery industries.

### Background of the tanning industry

As is well known, in order to be used effectively, natural animal pelts first need to undergo a series of rather complex treatments - generally summed up under the term "tanning" - by which the skin acquires properties of preservability, softness, mechanical strength, flexibility, and colour, which allow the skins to be currently used in a great number of applications in the industries listed above.

However, due to the nature and to the structure of the raw materials used, tanning processes have some important technological limitations, some of the main ones being the following:
- low yield of the process transforming the raw skin into collagene, which allows to exploit only 48-51% of the initial product and consequently produces large amounts of waste material (substantially consisting of collagene) which must be disposed of as solid waste;
- high levels of pollutants in the water and gaseous effluents of tanneries, which require the presence of suitable purifying treatments to prevent said pollutants from being released into the environment;
- low yield of the chemical process for the stabilisation of the raw materials. As a matter of fact, during the tanning process, an excess of chemicals is used (for one ton of raw skin about 80 kg of chrome salts, up to 450 kg of vegetal extracts and an average of 50 m³ of water are used), on account of the high compactedness of the raw skin and of the need to obtain diffusion of the chemicals inside the fibre structure thereof; furthermore, such amounts are calculated on the weight of the raw material, characterised by a high water content.

To increase the yield of raw skin processing, production of reconstituted skin from the collagene solid wastes produced during the processing of the skins has been suggested, through suitable chemical-physical treatments of said wastes. However, this choice did not prove to be a commercially viable option, both for the significant technical problems related to said treatments, and for the extremely low economic value of the obtained skins, as is highlighted by the values taken on by an indicator known as "economic exploitation potential" (equal to the ratio production cost/gain for unit of finished product) for this type of processes which reclaim collagene waste materials. It is clear in fact that, for an exploitation process of a certain material to be economically convenient, the economic exploitation potential must be lower than 1. On the contrary, the production of reconstituted skin from collagene-based waste materials has an economic exploitation potential decidedly greater than 1, which makes economically unviable a possible exploitation process of the tanning solid wastes, which is hence actually valued only as a possible method for disposing of said residues, alternative to other types of disposal but which in any case adds an economic burden to the main process.

Besides this, in recent years campaigns by animal activists on issues such as fur production and the ban on animal testing of cosmetic products, have led to the setting up of franchising chains selling only products which did not require the killing of animals (Miele, 1998), even coming to the suggestion of using "ethical skin", that is skin from animals who died of natural causes (mostly from diseases) and not because they were slaughtered. This trend could have a remarkable effect on the reduction of the market for natural leather and see a constantly rising number of consumers prefer, in the next future, "bioengineered" leather as an alternative to animal-origin leather.

From the picture of the prior art set forth above it can be noticed that the market clearly expressed two different needs: on the one hand, that of further improving the yield of traditional tanning processes simultaneously reducing their environmental impact, on the other hand that of offering to a - currently sharply rising - share of consumers sympathetic to animal issues a material having the same properties and features of natural leather, whose manufacturing process, however, does not involve the killing of animals.

It is hence an object of the present invention to meet these requirements through alternative materials and processes which may solve in one step the different problems illustrated above.

In particular, a first object of the present invention is that of identifying a manufacturing process of materials based on animal skin which allows to reuse the solid collagene wastes of the current tanning processes, by exploiting the latter in an economically viable manner.

A second object of the present invention is then to reduce the environmental impact of the polluting materials from tanning processes, both reclaiming the solid wastes of the current tanning processes, and providing new materials based on animal skin which require simplified tanning processes.

Finally, a third object of the present invention is to offer products based on animal skin which, however, meet the requirements of those consumers sympathetic to animals, being manufactured from the solid wastes of current tanning processes, or from "ethical leathers".

### Summary of the invention

Such objects are achieved, according to the present invention, by means of a bioengineered tissue of animal origin for industrial uses characterised in that it comprises a plurality of layers of bioengineered sheet material, obtained and interconnected through in vitro tissue-forming and/or tissue-repairing processes by fibroblasts seeded on bioactive scaffolds.

The invention also relates to the bioengineered sheet material obtained through in vitro tissue-forming and/or tissue-repairing processes by fibroblasts seeded onto bioactive scaffolds, as an intermediate product for the preparation of said tissue.

Finally, the invention relates to some preferred in vitro processes through which several layers of said bioengineered sheet material are interconnected through in vitro tissue-forming and/or tissue-repairing processes to obtain said tissue.

### Brief description of the drawings

The invention will now be described in further detail, with reference to some exemplary preferred embodiments of the same, illustrated also with reference to the accompanying drawings, wherein:
fig. 1 shows fibroblasts NIH-3T3, cultured on HYAFF, pretreated with medium (A) or with collagene (B) after a 17-day culture;
fig. 2 shows histological sections of HYAFF treated with collagene and seeded with fibroblasts NIH-3T3, after a 17-day culture. Coloured with hematoxylin/eosin. A) 40x; B) 20x;
fig. 3 shows NIH-3T3 cultured on collagene-based waste material from the tanning process (Split) bioactivated with medium (A) or with collagene (B);
fig. 4 shows a bioengineered sheet material from collagene-bioactivated splits, seeded with NIH-3T3 and cultured for 8 (A), 15 (B), and 18 (C) days. Coloured with hematoxylin/eosin;
fig. 5 shows bovine fibroblasts (FB) cultured on collagene-bioactivated splits. Phase contrast;
fig. 6 shows FB cultured on medium-bioactivated split, coloured with hematoxylin/eosin at 15 days from seeding. (A) 40x; (B) 60x;
fig. 7 shows FB cultured on medium-bioactivated split. Coloured with hematoxylin/eosin at 7 days from seeding. (A) 40x; (B) 20x;
fig. 8 shows NIH-3T3 cultured on collagene microparticles. Observation under a phase-contrast microscope;
fig. 9 shows NIH-3T3 cultured in collagene gel. Macroscopic observation;
fig. 10 shows FB cultured in collagene gel. Observation under the phase-contrast microscope, 40x;
fig. 11 shows microscope observations at 40x in two different areas of FB cultured on 8-month-old split, employing ascorbic acid and previously cobalt-sterilised for 20 minutes, after an 11-day culture;
fig. 12 shows two microscope observations at 40x of a culture similar to that of fig. 11, but without employing ascorbic acid and with a sterilisation time of 8 minutes;
fig. 13 shows microscope observations at 20x and 40x of a same area of NIH-3T3 culture on fresh split, without employing ascorbic acid and previously cobalt-sterilised for 8 minutes, after a 14-day culture; and
fig. 14 shows microscope observations at 20x and 40x of a culture similar to that of fig. 13, with a 15-minute sterilisation time;

### Background in the biomedical industry

From the summary of the invention reported above, it is clear that in the present invention biological techniques - themselves already widely developed in the biomedical industry - are largely employed concerning cell handling/reproduction and, more generally, *in vitro* tissue formation/repair. In the following a short overview will hence be given of the state of the art in this particular industry, for the purpose of diagrammatically illustrating available technologies for the application in the present invention.

It was demonstrated time ago that animal cells dissociated from their original tissue are capable of creating new tissues when cultured in vitro onto three-dimensional supports in an optimal environment. It has been shown to be possible, for example, to form tubular structures from dissociated endothelial cells (Montesano et al., J. Cell. Biol. 97:1648, 1983) or cartilage tissue from isolated chondrocytes (Kisiday et al., Proc. Natl. Acad. Sci U S A. 99:9996, 2002). In suitable chemical-physical conditions and in suitable conditions of nutrients feed, when the dissociated cells are cultured on a 3D scaffold representing a temporary mechanical support for the same, the cells are capable of reconstructing their typical tissue because they retain in vitro also their ability to produce extracellular matrix (ECM) (Webb et al., Biomaterials 24:4681, 2003), the "binding cement" material in which they are naturally immersed *in vivo.*

This cell property is exploited in tissue engineering, a new bioengineering frontier which - through the application of engineering and life-science principles - aims at achieving the *in vitro* regeneration of biological tissues, so as to replace damaged or malfunctioning tissues (Langer and Vacanti, Science 260:920, 1993). This new biotechnology hence consists in the culturing of specialised cells onto suitable biodegradable 3D polymeric scaffolds (biomaterials), to obtain in vitro tissue bio-hybrids. The classic scheme of tissue engineering applied to the medical field consists in removing a fragment of homologous tissue (for example skin, cartilage or bone of the patient onto which the new tissue will have to be grafted) from which the cells are removed through specific proteolytic enzymes; the dissociated cells are then grown and successively cultured onto specially-prepared biomaterials (scaffolds) so as to obtain production of a neo-tissue. It has been reported that, by suitably varying culture conditions, it is possible to stimulate biosynthetic cell activity, for the purpose of obtaining an extracellular material which is structurally and functionally similar to the original tissue.

Tissue engineering, some products of which are already used in clinical practice, has the potential of revolutionising the current knowledge of in vitro animal tissue regeneration. Some representative products, at various development stages, comprise products for the regeneration of skin, cartilage, ligaments and tendons, bones, up to complex organs such as heart, liver and pancreas.

For example, dermis replacements (for example a product known on the market under the trademark Dermagraft) are already in use in clinical cases. Such products are obtained by inserting human fibroblasts from newborns - generally removed during circumcisions, separated through enzymes and then amplified in culture - in a polymeric bioabsorbible support having a reticular structure. The cells adhere, spread and grow in the polymeric net in which they are cultured for 2-3 weeks. The fibroblasts inserted in the net, during their proliferation, secrete growth factors, collagene, fibronectin, glycosamminoglycans and other proteins creating the ECM and hence forming a functional tissue manufactured in vitro. After this first growth period in vitro the tissue is then transplanted in vivo for the final stage of development and of adhesion to the tissues surrounding the graft.

Another product at an advanced stage of development concerns the regeneration of cartilage, which can be reconstructed with a preset shape. The reconstructed cartilage represents a new goal over reconstructed skin, since its molecular structure is noticeably more complex. The procedure provides the growth of isolated chondrocytes in three-dimensional synthetic or natural matrixes. The matrix material is progressively fully replaced by cartilage tissue produced by the cells themselves (Vacanti et al., Mat Res Soc Symp Proc., 252:367, 1992).

An even further advanced example of the technological advancements in this field is the recreation of corneas from extremely small quantities of cells removed from the patient, which are then cultured and grown in vitro on thermosensitive supports. Currently, a number of bioengineering companies exist which have been marketing or are launching products and/or processes for repairing different tissues, such as: skin (Organogenesis), heart (Genetech), pancreas (BioHybrid Technologies), urinary apparatus (Integra LifeSciences), cartilage and bones (Osiris Therapeutics), breasts (Reprogenesis), liver (Advanced Tissue Sciences), (Arnst C. and Carey J., Business Week, July 27,1998) etc.

The principles of tissue engineering rest upon the combined and coordinated use of three main components which are illustrated more in detail in the following: scaffold biomaterials, cells, culture media and conditions.

### Biological materials

Biomaterials, whether natural or synthetic, play a fundamental role in *in vitro* tissue regeneration, since they represent the mechanic support of cell cultivation, define the size and shape of the future engineered tissue, spread cells in an accurate manner and guide the tissue-forming process (Wong and Mooney, in Synthetic Biodegradable Polymer Scaffolds A. Atala and D. Mooney Ed., 1997, Birkhauser, Boston).

To fabricate scaffolds for tissue engineering applications, diverse materials of natural origin (such as ialuronic acid and collagene) or synthetic origin (such as polyglycolic acid - PGA and polylactic acid - PLA) can be used. They must have specific features, such as bio-degradability, bio-compatibility, capability of interacting with cells by means of specific bonds, and have suitable mechanical and physical properties (porosity). In practice, biomaterials used as supporting scaffolds of a cell culture, for in vitro formation of a tissue, work as synthetic extracellular matrixes and their suitable design allows to mime the entire range of biological and mechanical functions of the original ECM.

To allow the formation and the development of a functional tissue, it is necessary to first condition the biomaterial which makes up the scaffold, so as to determine a good interaction between the cells and the material. The lack of a specific cell-scaffold recognition in fact jeopardises cell proliferation and thereby tissue formation. The importance of the biomaterial which makes up the scaffold is exemplified by the results obtained on monolayers of vicryl-cultured endothelial cells (Advanced Tissue Science, La Jolla, CA), which show how said biomaterial influences cell orientation and - reasonably - also cellular function (Nerem et al., in Synthetic Biodegradable Polymer Scaffolds A. Atala and D. Mooney Ed., 1997, Birkhäuser, Boston).

Fibrous polymeric scaffolds, for example, provide pores into which the chondrocytes can penetrate, and also a three-dimensional space and a mechanical support to host the adhered cells, as these grow and build an extracellular cartilage matrix on the scaffold polymer. This process determines the formation of new cartilage, whose structure and shape are determined by the similar features of the scaffold (Ma et al., J. Biomed. Mater. Res. 29:1587, 1995).

The knowledge acquired up until today concerning the biomaterials which may be used as scaffolds for cellular cultures and their role in the interaction with the cellular component allows to suitably engineer the polymeric matrixes which make up said scaffolds so as to guide and condition the regeneration and formation process of biological tissues.

Conditioning the material at issue with bioactive molecules such as those present in the culture medium has further been proved to be particularly effective for promoting the adhesion of the cells seeded on the scaffolding biomaterials (which adhesion is strategic for culture success, as will be explained in the following). Preconditioning of the material with adhesive molecules favourably mediates the initial stages of cell adhesion, favourably conditioning subsequent tissue development.

### Cells

Cells represent the second essential component of tissue engineering: they are in fact responsible for the formation and growth of the neo-tissue.

*In vitro* cell cultivation, widely used in molecular and cellular biology since about thirty years ago, is a simple technique of inestimable value, given the possibility of widening at will the starting cell population (especially if such population has "tumoral", or better, "transformed" features), of freezing it when necessary (to preserve it, even for long periods), and finally to defrost it when required. It has allowed the study of physiological and pathological cell processes.

### Culture conditions

To be able to grow outside a living organism (*in vitro),* cells must be placed in strictly controlled conditions, recreating as best as possible those present *in vivo.* It is by now consolidated knowledge that cells must first of all be able to adhere to a solid substrate: in conventional cultures, cellular adhesion and growth occurs onto surfaces (bidimensional or 2D cultures); more recently, protocols for cultures on three-dimensional (3D) supports have been devised, already termed above as "scaffolds". This has represented an improvement of culture conditions and results, since a situation more similar to that present in vivo is reproduced, which allows the *in vitro* production of tissues (or similar).

It is further known that cells require optimal temperature, osmolarity and pH conditions, and nutrient supply. Given that different cell types may have different nutritional requirements, various types of culture media and of sera for specific requirements are by now available on the market. Furthermore, there is a constantly growing number of identified biomolecules (growth factors, cytokines and vitamins) which, when added to the culture medium, may induce specific cell functions. For example, the in vitro production of extracellular matrix by fibroblasts may be increased adding ascorbic acid (Freiberger et al., J. Invest. Dermatol. 75:425, 1980). Endothelial differentiation may be stimulated by adding VEGF (Vascular Endothelial Growth Factor), and bone differentiation by the BMP-4 (Bone Morphogenetic Protein-4) protein. In other words, current knowledge allows to be more and more accurate in directing and stimulating cellular differentiation in the desired manner.

Furthermore, it is also known that the mechanical interactions to which a cell is subject affect its structure and function (Nerem et al., Am. J. Med. Sci. 316:169, 1998). In particular, hydrodynamic culture conditions may affect in vitro tissue formation in at least two ways: due to a direct action of the hydrodynamic forces onto cellular morphology and function, and due to an indirect action, mediated by the flow, on the exchange of nutrients and metabolites (Vunjak-Novakovic et al., Biorheology, 39:259, 2002).

A technologically limiting aspect for the manufacture of an *in vitro* tissue which is economically viable from an industrial point of view is represented by the need to produce ECM components (collagene, elastin, etc.) in a relatively short time, which is not necessarily compatible with the normal biosynthetic activity of cells. This technological limitation may be overcome by correctly using genetic engineering techniques which, as known, allow to increase the quantity and to favourably modify the type of extracellular matrix produced, thanks to genetic manipulations of the culture cells.

It can hence certainly be said that the scientific and bioengineering potential of cellular cultures has dramatically increased thanks to the appearance of genetic engineering: this technology has in fact allowed the introduction of foreign genetic material into cells, making it possible to manipulate different types of cells both for research purposes and for various types of biotechnological and biomedical applications. Genetic engineering is by now an easily exploitable technology, so much so as to be applied to extremely varied fields such as, for example, the agricultural and food industry (where it has led to the introduction of genetically "improved" products, which have properties that cannot be obtained through standard selection and breeding techniques (Ramón D. I geni che mangiamo, Ed. Dedalo, 2000 - The genes we eat), and clinical medicine (with the application of the so-called "gene therapy").

Tissue engineering, too, has recently started to employ genetic manipulation techniques: it has been proved, for example, that the introduction of human genes into staminal mesenchymal cells causes improved bone and cartilage regeneration (Mason et al., Clin. Orthop. (379 Suppl): S171, 2000); again, the introduction of the gene for the angiogenic factor VEGF into hepatocytes transplanted into collagene beads in the peritoneum of mice causes a dramatic increase of capillary vessel formation and a significant increase of the growth of hepatocytes (Aijoka et al., Hepatology, 29:396, 1999). Therefore, the introduction of foreign genetic material into cells makes it possible to attribute new properties or to modulate those already existing, according to users requirements. Such properties, introduced in the form of genetic material, are later steadily transferred to the population deriving therefrom.

The introduction of suitable genes may therefore stimulate the synthesis of matrix components, noticeably reducing the time necessary for tissue regeneration. Furthermore, through genetic engineering it is possible to direct the synthesis towards specific components of the extracellular matrix. This opportunity opens the perspective of engineering bio-hybrid tissues with properties (for example elasticity) which may be set at the beginning, through a suitable modulation of the ECM components in the final tissue. While this opportunity is of little account in biomedical applications, it becomes instead an interesting perspective in the engineering of *in vitro* bioengineered tissue, in accordance with the teachings of the present invention, since it may allow the production of a tissue with optimal commodity properties depending on the specific application purpose. For example, in the applications of more immediate interest of the present invention, i.e. those replacing animal pelts of natural origin, in the fields of wear and furnishings, bioengineered tissues could be developed, which have improved deformability and breathability features.

### Detailed description of exemplary embodiments of the invention

The application of the cell culture technique for the production of a stabilised bioengineered tissue suitable for the replacement of cattle pelts has never been experimented before. Tissue engineering knowledge, as a matter of fact, has always been aimed at biomedical applications, in which on the one hand the origin, purity and specificity towards the individual patient/organ of the cells used is essential and, on the other hand, the tissue is cultured *in vitro* up to an intermediate development stage, sufficient to keep its vitality after the in vivo transplant, where the final development and formation of the finished tissue effectively occurs. However, the last years of research in this field and the specific research carried out by the inventors for an industrial application of this technology, has allowed to confirm the possibility of industrially obtaining *in vitro* bioengineered tissues in a finished form, i.e. tissues which may be used for a direct industrial use, without any *in vivo* passage, for example in the tanning industry.

One of the major obstacles which the inventors of the present invention had to face was that of obtaining finished bioengineered tissues with a thickness suited to the successive industrial applications for which such tissues were intended. For example, in the applications of such tissues in the tanning industry, it is essential to obtain a minimum thickness of 0.5 mm, and preferably above, up to 1-1.3 mm. Such a thickness may not be obtained through currently available cellular culture techniques, essentially due to the difficulty in spreading the nutrients and maintaining the optimal chemical-physical conditions for cell proliferation through said tissue thickness.

According to a fundamental intuition of the present invention, this obstacle has been overcome through a manufacturing process of the bioengineered tissue occurring in two different stages. In a first stage, a product with a small thickness is prepared, which in the following will be called "bioengineered sheet material" whether such material has formed on flat or on particle supports. In both cases the thickness of the bioengineered sheet material is such as to be apt to be obtained in a single cell culture session, and ranges for example between 50 and 200 µm.

In a second stage of the process, a plurality of said bioengineered materials in sheets are arranged in close mutual proximity and in cell culture conditions, so as to have a process of tissue repair which determines the biological union of the different layers or of the individual particles, thus forming the final bioengineered tissue in the desired thickness and shape.

According to the present invention, the bioengineered sheet material is formed by seeding fibroblasts onto three-dimensional bioactive scaffold materials, both natural and synthetic ones. The shape of such scaffolds can of course vary at will; the shapes currently considered to be preferable are the planar one, to obtain whole sheets of bioengineered material which are detached from the support at the end of the culture, or that of a particle material to obtain bioengineered material of a generally spherical shape which incorporates the scaffold biomaterial inside.

The scaffolds which may be currently employed essentially consist of the following materials:
scaffolds available on the market;
collagene waste material from tanning processes (split);
particles of collagene waste material;
microparticles of pure collagene.

Among the scaffolds available on the market, HYAFF, a semisynthetic derivative of ialuronic acid (HA), has been successfully used for the production of skin replacements for clinical applications (US-A-6110208).

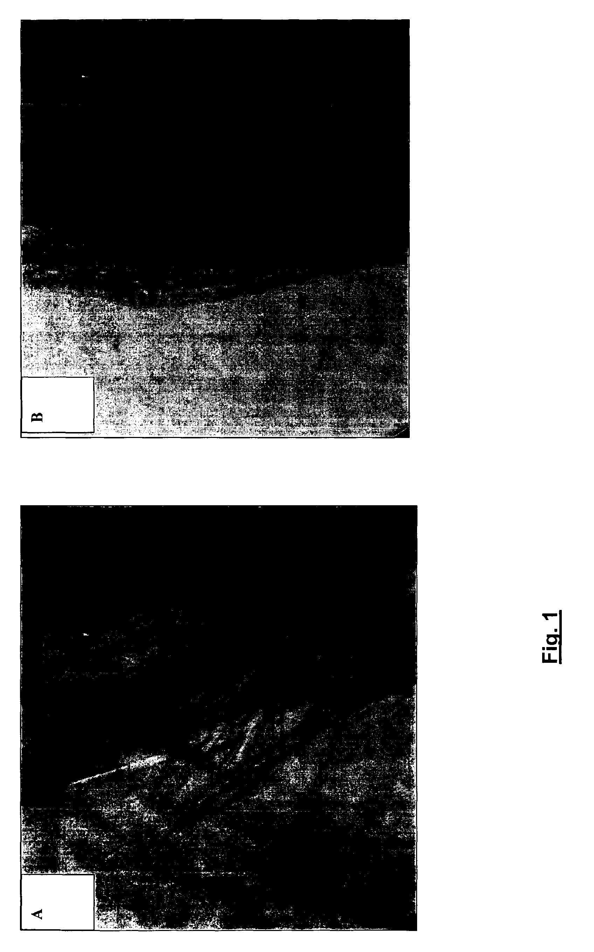

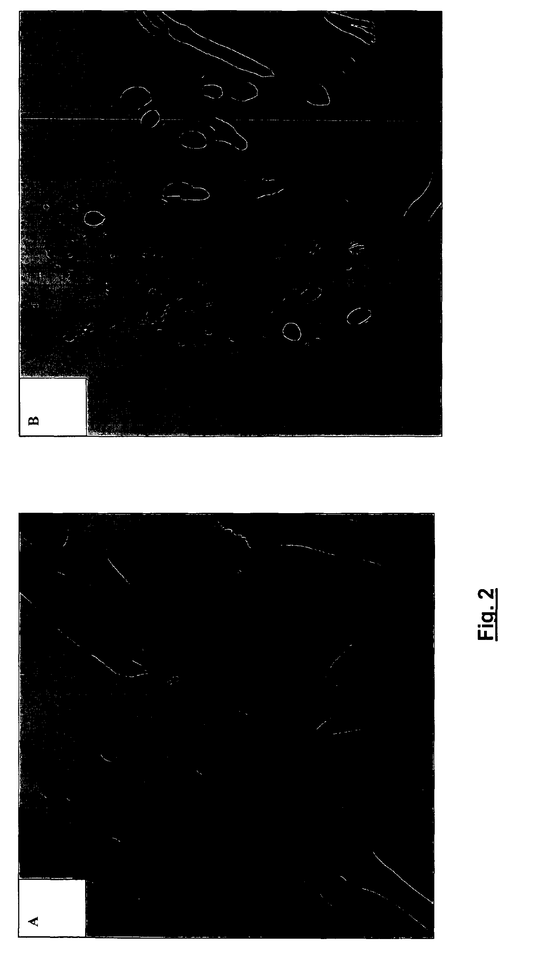

In accordance with what has been described in US-A-6110208, HYAFF can be bioactivated by collagene-incubation, to improve its adhesive properties (figs. 1 and 2). This makes the added cells capable of interacting and remodelling this same material.

Alternatively, collagene waste material from the tanning process in the form of sheets or particles is used as a scaffold for the growth of the neo-tissue. This material requires sterilisation before use to prevent the development of bacterial contamination which may be harmful to the cellular cultures. Sterilisation is performed with ethanol treatment and successive wash with PBS 1x.

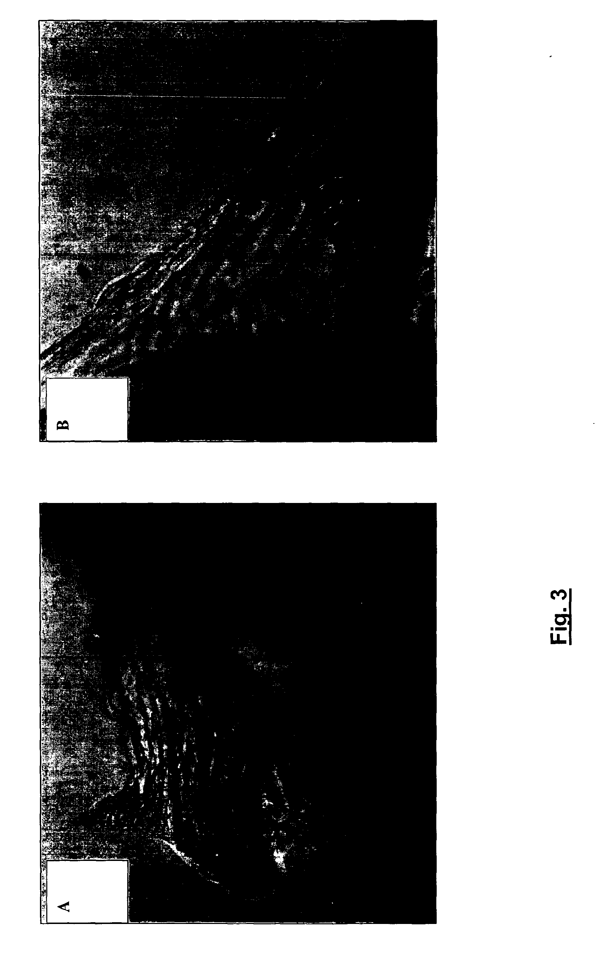

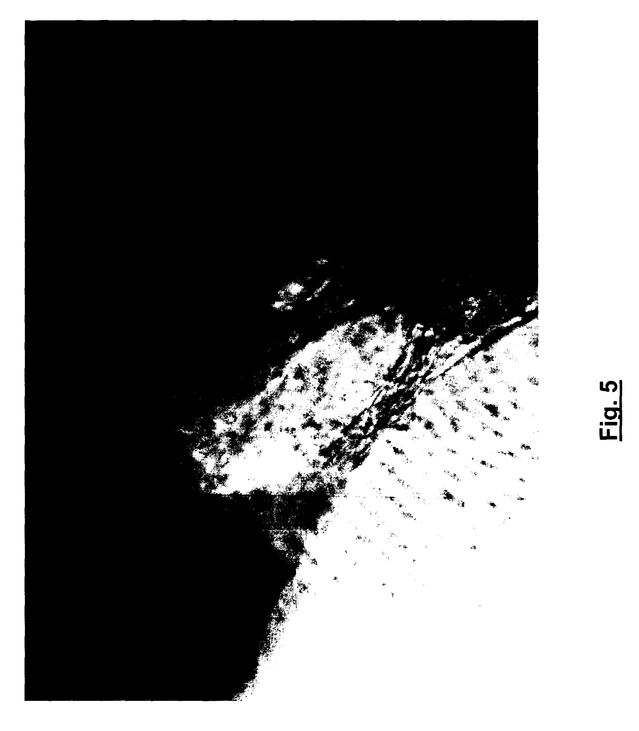

As in the case of HYAFF, collagene bioactivation can heighten the adhesion and repair capability of the cells (figs. 3 and 5).

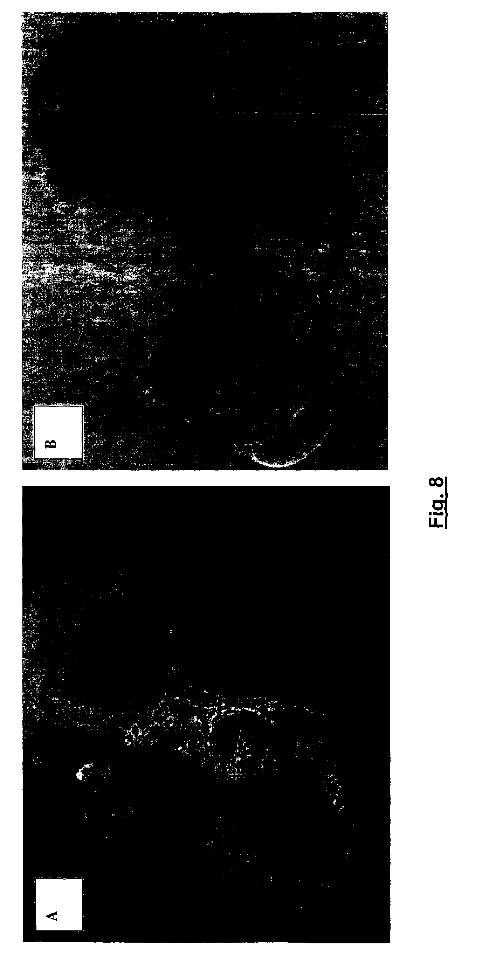

Finally, even a particle collagene material, from wastes or in pure protein form, can provide adhesion sites for the cells and thereby represent a supporting scaffold therefor. These cells, following the production of extracellular matrix and the establishment of cell-to-cell, cell-to-material and cell-to-extracellular matrix contacts, induce self-assembly and repair of the material, from particle to whole material (fig. 8).

The fibroblasts can be derived from cattle or mice. Murine fibroblasts (NIH3T3) have the following advantages:
they are capable of quick proliferation;
they are immortal;
they synthesise components of the extracellular matrix.

They are cultured in Dulbecco's modified Eagle's medium (DMEM), 10% of foetal bovine serum (FBS), 100U/ml of glutamine and 100U/ml of penicillin/streptomycin.

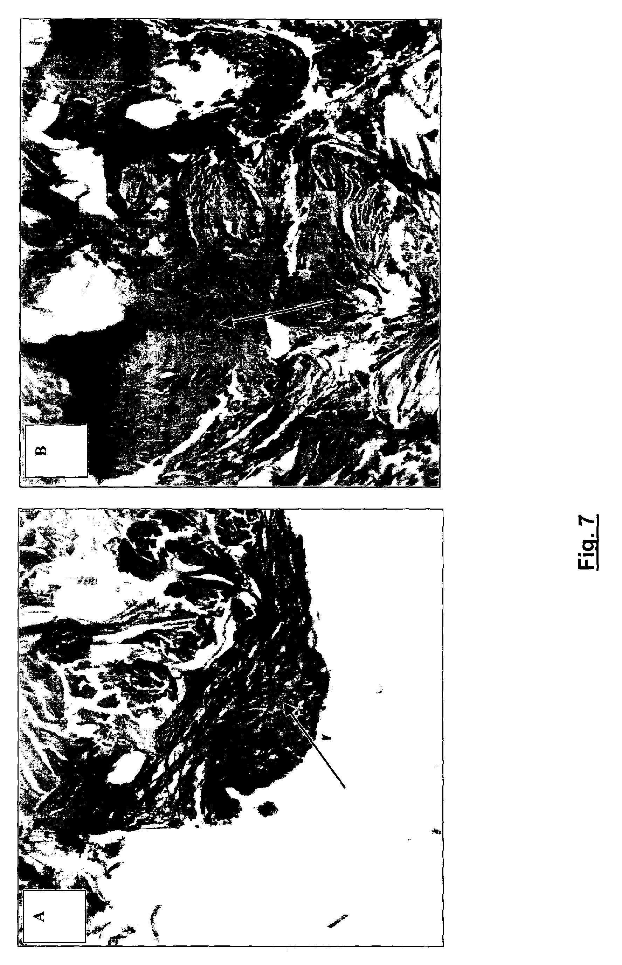

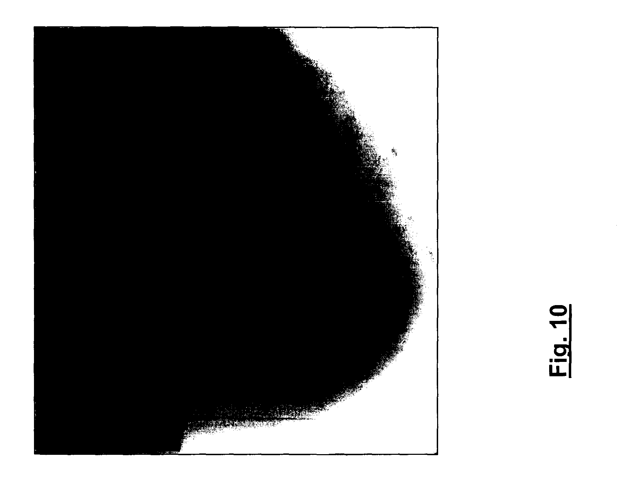

Unlike murine fibroblasts, bovine fibroblasts (FB: AG10385) (Coriell Institute, Camden, NJ) have limited proliferation capabilities. However, they have the following advantages:
given their origin, they are more suited to the application suggested in the present invention;
they have wide remodelling capabilities (figs. 5 and 10);
they synthesise a substantial extracellular matrix (Fig.7 A) .
they actively invade three-dimensional scaffolds (Fig. 7 B) .

Bovine skin fibroblasts are cultured in MEM Eagle-Earle BSS enriched with a solution of essential and non-essential aminoacids (2x), 20% of FBS, and glutamine 2 mM.

This cellular line further has a varying number of chromosomes. This may lead to the selection of an immortal population within the original one; such capability is extremely important, due to the possibility of having immortal cells available, which may therefore be perpetuated in a production-aimed process.

By using genetic engineering techniques, the fibroblasts may be genetically modified so as to:
be immortal;
induce the synthesis of larger quantities of specific molecules of the extracellular matrix (collagene, elastin, etc.);
attribute peculiar properties to the finished product (for example greater elasticity, greater strength, etc.).

Since immortalisation of differentiated cells, such as fibroblasts, can lead to loss of differentiation, with resulting loss of the cell biosynthetic capacities (including synthesis of extracellular matrix proteins), "stabilisation" of FB can be performed by the expression of a temperature-sensitive mutant of the T antigen of the SV40 virus. At the permissive temperature of 33°C, the cells will behave as immortalised cells (Nabi et al., J. Cell. Sci. 104:37, 1993; Kilty et al., J. Neurochem. 73:1859, 1999); at the non-permissive temperature, they will behave instead as differentiated fibroblasts, retaining their capacity for synthesising extracellular matrix.

The capacity to synthesise matrix can be further stimulated by the administration of factors such as ascorbic acid (50 µg/ml).

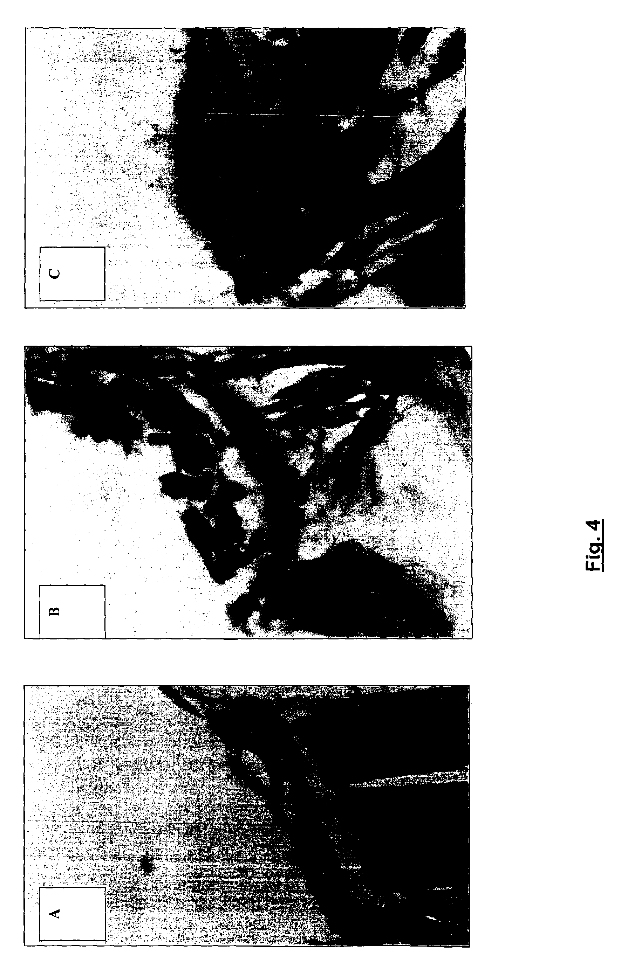

After having reached the semiconfluence stage, the cells are trypsinised and 2x10⁵ - 4x10⁵ cells/cm² are seeded on the scaffolds. The adhesion capacity depends both on the type of cell and on the possible bioactivation of the material. The cells, once they have adhered to the fibres of the substrate, establish relationships with other nearby cells applying a tensile force on the same and consequently remodelling the substrate itself (Fig. 3). Interaction with the material is very quick and the formation of neo-tissue may be detected already after 7-8 days (figs. 4 and 7).

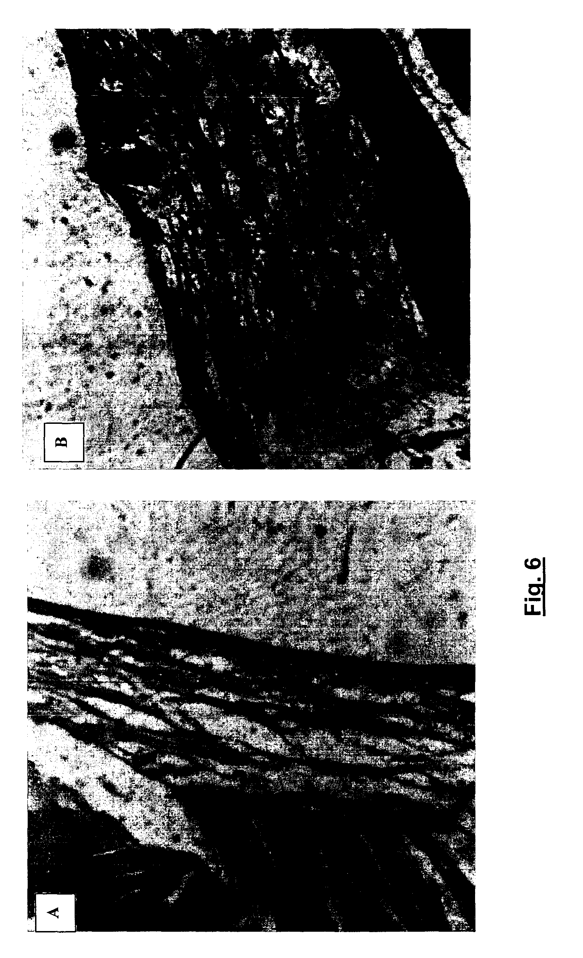

The quantity of matrix produced depends on the cellular type, on the culture conditions (including the presence of factors stimulating the production thereof), on the nature of the substrate, and on the number of plated cells: for example, FB give a more abundant matrix when 4x10⁵ cells/cm² are seeded on the scaffold, compared to when 2x10⁵ cells/cm² are seeded (please compare fig. 6 and fig.7).

Collagene bioactivation induces NIH-3T3 fibroblasts to form a thicker neo-tissue.

If necessary, the bioengineered sheet material is transferred onto new plates, to prevent the cells which have adhered to the plate from reaching confluence.

By means of histological analyses (colouring with hematoxylin-eosin the bioengineered material sections) the evolution over time of the neo-tissue can be observed (figs. 2, 4). The samples, once collected, are fixed in formalin (24 h), sequentially dehydrated in alcohol (75°, 85°, 96°, 100°) and embedded in paraffin. Sections of 5 µm each are made by (Zeiss) microtome cut and subject to morphological analysis by colouring with hematoxylin/eosin.

In the following, examples are given which show growth stages in the formation of the bioengineered sheet material of the present invention. Such examples are set forth here as a mere illustration of the invention and must hence not be interpreted as limiting the same.

### Example 1

Firstly, NIH-3T3 cells have been amplified in 100-mm Petri plates.

200,000 cells have been seeded onto HYAFF, upon incubation in culture medium, gelatine or collagene. The incubation of HYAFF in collagene aids not only fibroblast adhesion to the scaffold, but also the acquisition of a physiological shape, the capability of extending onto distant fibres determining remodelling of the biomaterial in respect of a HYAFF incubated with gelatine or culture medium (figs. 1 and 2) and matrix production (figs. 2 and 4). The scaffold remodelling effected by the fibroblasts and aided by preincubation in collagene is apparent upon direct microscopic observation, where the material is completely flattened out, lacking the roughness which characterises the original material (Fig. 1).

Histological analyses with hematoxylin/eosin have revealed the presence, already after 9 days, of an ECM layer mixed with cells on the surface, which is thicker in the collagene-activated materials, than in the medium-activated ones. After 17 days the cells appear to have invaded the inside of the scaffold, too, developing an evident matrix layer (Fig. 2).

### Example 2

200,000 NIH-3T3 cells have been seeded onto collagene waste material of the tanning process, upon incubation with culture medium or collagene.

This material requires sterilisation before use to prevent bacterial contamination, which is harmful to the cell cultures. Sterilisation is performed through treatment with ethanol and successive washing with PBS 1x.

Direct microscopic observation highlights the adhesion and remodelling capacity of the cells, aided by pre-incubation with collagene (fig. 3).

Histological analysis identifies a thin layer of cells along the scaffold edges together with a similarly thin layer of ECM. In the case of collagene-bioactivated split, the matrix appears to be thicker (Fig. 4). With this type of analysis we have followed over time the evolution of the neo-tissue, showing its growth and maturation (fig.4).

### Example 3

200,000 fibroblasts FB are seeded onto collagene waste material. Bovine fibroblasts, as well as murine ones, interact with the material, remodelling it (Fig. 5). FBs have a greater adhesive and remodelling capacity than NIH-3T3 fibroblasts, so that scaffold bio-activation can be unessential or made up by medium only. Histological analyses show the formation of a continuous surface germ layer mixed with cells, which in some cases is even rather thick (fig. 6). When a larger number of cells (3x10⁵) is seeded, the formation of a thick neo-tissue is observed, not only on the surface (fig.7 A), but also inside the scaffold (fig. 7 B), revealing a widespread cell migration with successive matrix synthesis.

The cells prove capable of repairing the scaffold structure, invading the spaces thereof. This observation suggests that it is possible to reproduce a bioengineered tissue *in vitro* from waste material, exploiting the capacity of the cells to repair the substrate.

### Example 4

Collagene microbeads have been used as a substrate onto which NIH-3T3 fibroblasts have been cultured.

The cells first adhere to the collagene particles. After a few days, fibroblasts adhered to the particles join cells of adjacent particles, drawing them nearer (fig. 8A), with the resulting assembling of the particled substrate (fig. 8B). Matrix synthesis by the fibroblasts can ease such process. The presence of ascorbic acid can stimulate such assembling by increasing ECM production. Such property can be exploited for the creation of new tissue.

### Example 5

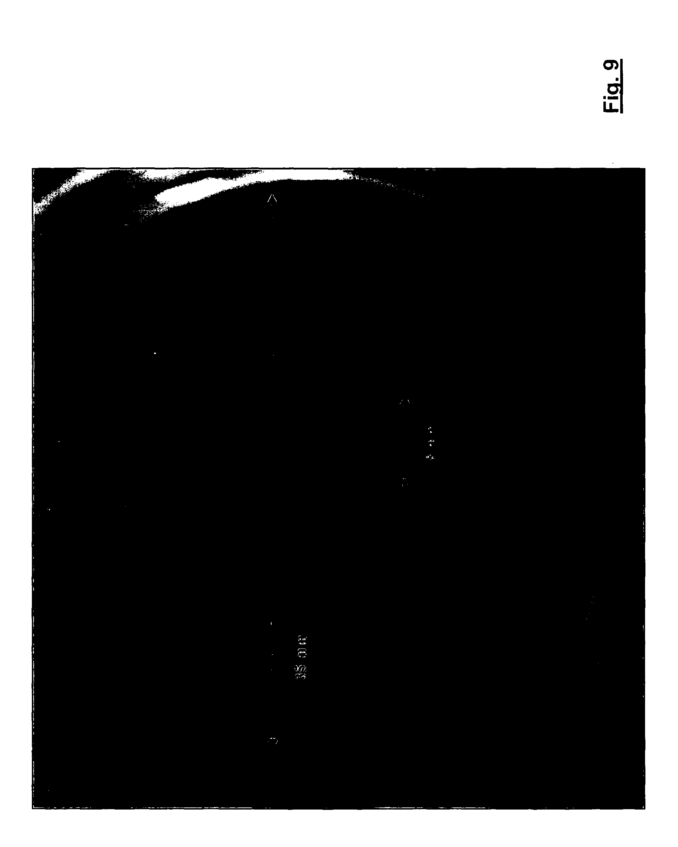

NIH-3T3 fibroblasts have been trypsinised and 100,000 cells/ml have been resuspended in collagene (1.2 mg/ml), in the presence of DMEM + 10% FBS culture medium and have been plated on 35-mm, low-adherence plates. The fibroblasts create bonds with the collagene fibres, generating tensile forces which, after a few days, lead to the contraction and remodelling of the collagene scaffold. As can be seen in fig. 9, the scaffold - which at the beginning of the culture occupies the whole surface of the plate (35-mm diameter) - is reduced by about 80% (8-mm diameter) (Fig. 9). This result shows the capacity of fibroblasts to perform macroscopic modifications reorganising and remodelling the substrate.

### Example 6

100,000 fibroblasts FB/ml have been resuspended in collagene (1.2 mg/ml) in the presence of culture medium onto 35-mm, low-adherence plates. As observed for NIH-3T3 fibroblasts, the fibroblasts FB are capable of contracting the collagene within a few days (fig. 10). However, bovine fibroblasts display a collagene-contracting capacity exceeding 90%, proving that different cells have different scaffold-remodelling capacities and that non-immortalised cells - still expressing typical differentiation features - may represent a more effective system for "repairing" a neo-tissue. Furthermore, these results show that the opportunity to select cells with different repair capacities may represent a useful variable, which may be modulated according to user requirements.

### Example 7

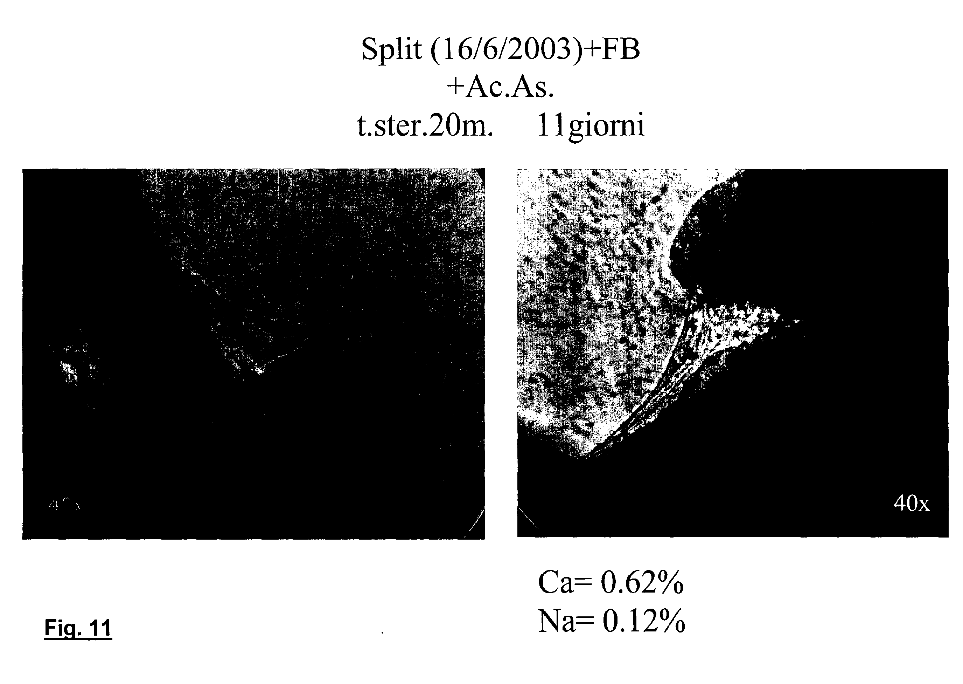

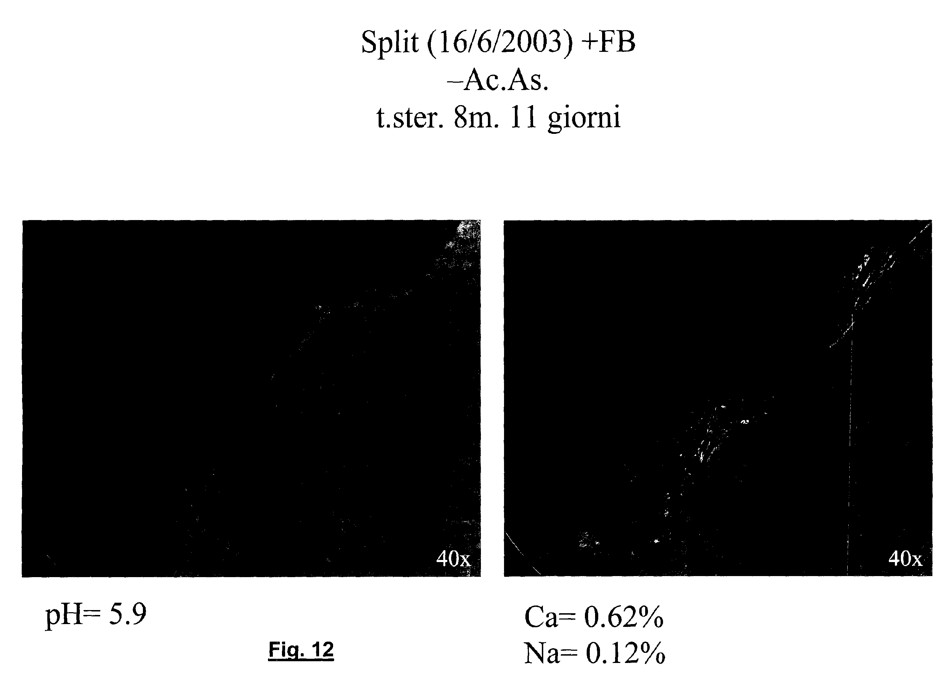

Split collected from tanning processes which took place about 8 months before the experiment has been used as scaffold. Before seeding with bovine fibroblasts FB, the split has been sterilised with cobalt for different times of 8 and 20 minutes and one of the two cultures has been treated with ascorbic acid (figs. 11 and 12) .

From the study of the microphotographs - taken in both cases after an 11-day culture - it can be seen how, independently from the sterilisation time, with and without ascorbic acid, the bovine fibroblasts have perfectly adhered to the scaffold and grown, remodelling the various indentations initially displayed by the material.

### Example 8

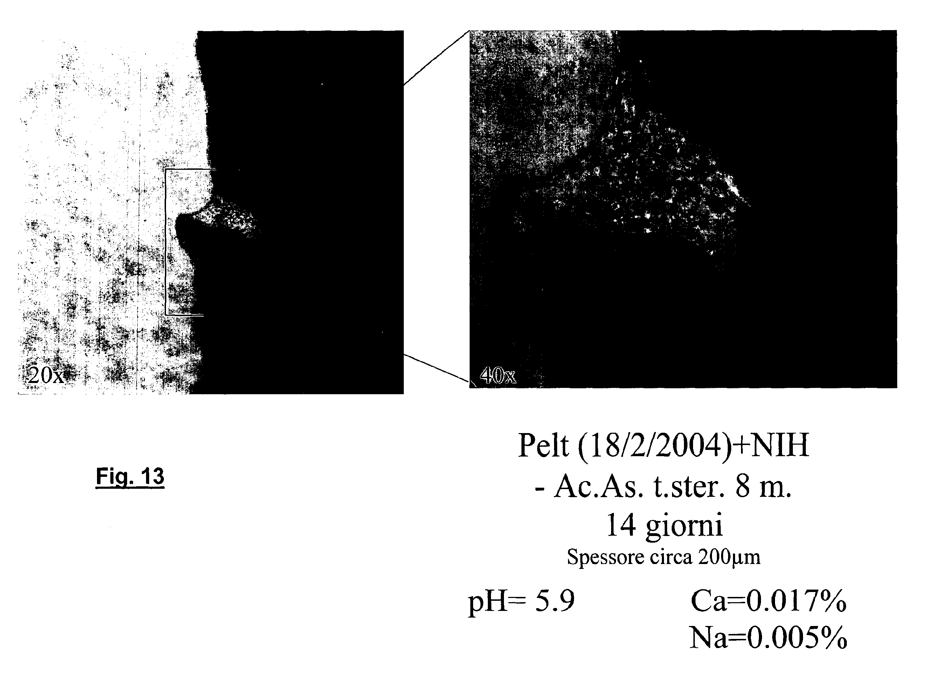

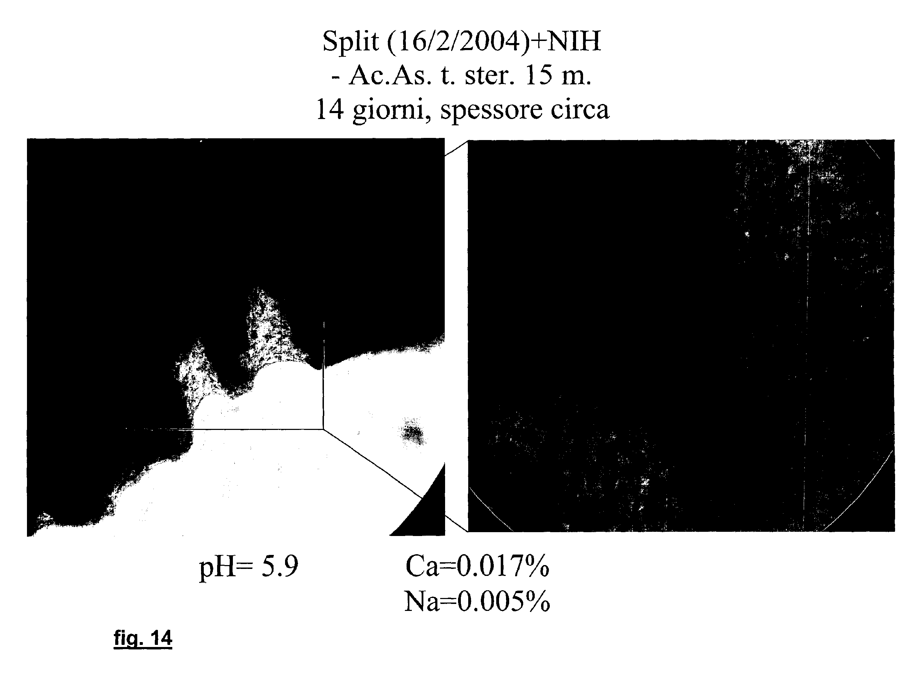

The experiment was performed in a similar way to that of Example 7, using, however, "fresh" split collected from tanning processes just carried out, which displayed sodium and calcium concentrations decidedly smaller than the split used in Example 7. In this case, cell seeding has been carried out with stable murine NIH-3T3 fibroblasts and the split has been sterilised with cobalt for different times of 8 and 15 minutes; neither of the two cultures has been treated with ascorbic acid (figs. 13 and 14) .

From the study of the microphotographs - taken in both cases after a 14-day culture - it can be seen how, independently from the sterilisation time, murine fibroblasts also have perfectly adhered to the scaffold and grown, filling and remodelling the various indentations initially displayed by the material.

Cell growth in this case has developed for a remarkable thickness of about 200 µm; it has been possible to detect that cell growth occurs both on the surface and inside the newly-formed bioengineered material.

These last two Examples prove that the invention is applicable, without significant differences in the degree of proliferation, to split of different origin, production age, and sodium and calcium content; even sterilisation times do not appear to provide different results, so that even shorter sterilisation times may be considered optimal.

The above-illustrated Examples have proved that it is possible to produce neo-tissue from bio-material and fibroblasts. Fibroblasts, through a repairing process, can rebuild the material within which they are cultured. Their repair capacity can be boosted by introducing specific genes and can be exploited for the growth of bioengineered materials and tissues.

From the description and from the above-illustrated Examples, it is also evident how the present invention has achieved the preset objects, providing a new protocol which comprises to culture cells onto bioactive materials, in the form of sheets or particles, for the purpose of producing a dermis-like bioengineered tissue to be used for industrial purposes and in particular in the tanning and pelts industry. The sheets or the particles can be made of a synthetic biomaterial or of collagene material directly deriving from residues of the tanning process.

Said protocol comprises:
a three-dimensional scaffold made of a bioactive material, capable of interacting with the cells;
the previous sterilisation of said scaffold and its possible bioactivation;
the amplification of (bovine or murine) skin fibroblasts in standard cell cultures;
the possible genetic modification of such fibroblasts, to make them immortal and/or to boost their repair capacities;
the seeding of suitably chosen and cultured skin fibroblasts on the above-mentioned scaffold;
the culture conditions for the optimisation of the bioengineered tissue production.

Thanks to the capacity of fibroblasts to adhere to the support provided, to grow and produce new extracellular matrix, new tissue can be produced endlessly.

In case particled material is used, the capacity of the cells to reassemble a whole tissue from particled material is exploited. Such process can be considered as a "repair" operation of the collagene waste material, effected by suitably specialised cells.

The neo-tissue, recreated as here described, can be employed as a new raw material in industry and in particular in pelt production.

The regeneration and repair process used for the production of the bioengineered tissue of the present invention guarantees mass and volume minimisation of the solid and liquid wastes, and above all improvement of their hygiene and quality features. It further allows to obtain the revaluation and reuse of selected products from solid wastes and from waste pelts deriving from the current tanning processes, or to use as starting material "ethical skins" only, thereby achieving all the objects of the present invention. The bioengineered tissue according to the present invention hence represents a possible revolution of the current tanning process, not only due to the different origin of the raw material, but especially due to the opportunity to reuse the waste products of said process.

## Claims

1. Bioengineered tissue of animal origin for industrial uses **characterised in that** it comprises a plurality of layers of bioengineered sheet material, obtained and interconnected through in vitro tissue-forming and/or tissue-repairing processes by eukaryote cells seeded onto bioactive scaffolds.

2. Tissue as claimed in claim 1), wherein said eukaryote cells are bovine or murine fibroblasts.

3. Tissue as claimed in claim 1), having a thickness above 200 µm.

4. Bioengineered sheet material obtained through in vitro tissue-forming and/or tissue-repairing processes by eukaryote cells seeded onto bioactive scaffolds having composition and morphology similar to animal dermis, as an intermediate product in the preparation of a bioengineered tissue as claimed in claim 1) .

5. Bioengineered sheet material as claimed in claim 4), having a thickness ranging between 50 and 200 micron.

6. Bioengineered material as claimed in claim 4), wherein said bioactive scaffolds are sterilised before seeding said cells.

7. Bioengineered material as claimed in claim 4), wherein said bioactive scaffolds are in the shape of particles.

8. Bioengineered material as claimed in claim 4), wherein said bioactive scaffolds comprise wastes from conventional pelt tanning processes.

9. Bioengineered sheet material as claimed in any one of the claims from 4) to 8), wherein said eukaryote cells are bovine or murine fibroblasts.

10. Process for making a bioengineered tissue as claimed in any one of the claims from 1) to 4), **characterised in that** several elements of said bioengineered sheet material are arranged adjacent in a same culture bed and are biologically interconnected through in vitro tissue-forming and/or tissue-repairing processes by eukaryote cells seeded in said culture bed.
